# EUROPEAN PATENT APPLICATION

(11) **EP 3 391 889 A1**
(43) Date of publication of application: **24.10.2018**
(21) Application number: 16876081.7
(22) Date of filing: 16.12.2016
(51) Int. Cl.: A61K 31/635, A61K 9/08, A61K 9/00, A61K 47/34

(54) **COMPOSITION, FOR OSTEOARTHRITIS TREATMENT, COMPRISING HYDROPHILIZED SULFASALAZINE AND HYALURONIC ACID AND METHOD FOR PREPARING SAME**

(30) Priority: 18.12.2015 KR 20150182260
(71) Applicant: BMI Korea Co., Ltd, Jeju-do 63309 (KR)
(72) Inventor: KIM, Suhwan, Ansan-si Gyeonggi-do 15491 (KR); MIN, Kyeong Woo, Asan-si Chungcheongnam-do 31471 (KR); BAIK, Yeong Jun, Seoul 06339 (KR); LEE, Sung Hee, Seongnam-si Gyeonggi-do 13603 (KR); WOO, Koo, Jeju-si Jeju-do 63093 (KR); KIM, Min-Kyoung, Jeju-si Jeju-do 63227 (KR)
(74) Representative: Hoppe, Georg Johannes
(86) International application number: PCT/KR2016/014822
(87) International publication number: WO 2017/105139

(57) **Abstract**

The present invention relates to a composition for treating osteoarthritis and a method for preparing the same, and more specifically, relates to a composition comprising hydrophilized sulfasalazine as an active ingredient and hyaluronic acid so as to enable effective treatment of osteoarthritis such as degenerative osteoarthritis by preventing joint wear in addition to symptom relief through intraarticular or local administration, and a method for preparing the same. When such a composition for osteoarthritis treatment is prepared by the preparation method according to the present invention, the dissolved state is maintained beyond a neutralization step after dissolution, preventing precipitation of sulfalazine by salting out occurring after neutralization , and thus a composition comprising high concentration of sulfasalazine can be prepared, and the composition prepared accordingly is highly effective in treating osteoarthritis, particularly degenerative osteoarthritis, through the intraarticular or local administration, since it comprises high concentration of sulfasalazine which had to be formulated at low concentration by conventional method due to poor solubility.

## Description

### [TECHNICAL FIELD]

The present invention relates to a composition for treating osteoarthritis and a method for preparing the same, and more specifically, relates to a composition comprising hydrophilized sulfasalazine and hyaluronic acid as active ingredients so as to enable effective treatment of osteoarthritis such as degenerative osteoarthritis by preventing cartilage degeneration in addition to symptom relief through intraarticular injection and local administration, and a method for preparing the same.

### [BACKGROUND ART]

Osteoarthritis refers to a state in which intraarticular cartilage is continuously degraded and damaged by cytokines and cartilage destruction enzymes expressed from chondrocytes and synovium through physical stimuli of articular region, resulting in pain, instability of joint and loss of mobility.

Currently, other than surgical operations, several medications, intraarticular (into synovial cavity) administration of hyaluronic acid, etc. are used for the treatment of osteoarthritis, but these are mainly intended for symptom relief and have little effect on cartilage damage reduction. Thus, drugs which can effectively treat osteoarthritis substantially have been in demand.

Meanwhile, sulfasalazine is currently used as an oral therapeutic agent of rheumatoid arthritis and inflammatory bowel disease, but the results of several recent studies reported that there is a possibility for sulfasalazine to be used for the treatment of osteoarthritis by protecting the change of chondrocyte, which is the cause of osteoarthritis, and inhibiting the expression of cartilage destruction factors from synovial membrane (Nose M. et al. J Rheumatol 1997;24:550-4, Lakey et al. Rheumatology 2009;48:1208-1212, Endo W. et al Mod Rheumatol 2014;24(5):844-50).

It is known that when sulfasalazine is taken orally, its absorption rate is very low as the blood concentration is 12 µg/ml even after the maximum dose of 4 g/day, and it also causes disgestive disorder due to poor solubility, and it has been reported that various side effects are caused by high blood concentration of sulfapyridine, which is a degradation product of sulfasalazine (Hoult JRS. Drugs 1986; 32:18-26). Therefore, direct intraarticular injection of sulfasalazine is expected to avoid those side effects caused by oral administration. However, poor solubility of sulfasalazine prevents liquid formulation with sufficient concentration for anti-osteoarthritis effect to manifest. In addition, since the turn-over time of synovial fluid is known as 2 hours (Levick JR et al. Ann Rheuma 1995; 54:417-423), intraarticular administration of sulfasalazine is not expected to be effective enough.

On the other hand, hyaluronic acid is one of major constituent of synovial fluid in the body and presents between articular cartilages to play a role of lubricant. As the concentration of hyaluronic acid in the joint is reduced in case of osteoarthritis patients, hyaluronic acid supplement is widely used as the osteoarthritis therapeutic material. Different from other component of synovial fluid, the half-life of the hyaluronic acid is more than 13 hours (Brown TJ et al. ExpPhysiol 1991:76; 125-134). Therefore, when sulfasalazine and hyaluronic acid are mixed, it is expected that residence of sulfasalazine in synovial cavity can be maintained for 24 hours or more.

Under these circumstances, in an effort to create an effective treatment method for osteoarthritis using sulfasalazine which is poorly soluble, the present inventors developed a pharmaceutical composition comprising high concentration of sulfasalazine as an active ingredient, which is dissolved with a specific concentration of basic additive, mixed with hyaluronic acid. Furthermore, as a result of applying the composition containing a high concentration of sulfasalazine and hyaluronic acid into an animal model, it is confirmed that the composition with a specific concentration level of sulfasalazine not only inhibits the progress of osteoarthritis, but also shows pain control at the same time, thereby completing the present invention.

### [DISCLOSURE]

### [TECHNICAL PROBLEM]

To solve the problem above, the present invention intends to provide a composition for treating osteoarthritis capable of intraarticular or local administration, comprising hydrophilized sulfasalazine as an active ingredient and additionally hyaluronic acid, salt or derivative thereof.

In addition, the present invention intends to provide a preparation method of the composition for treating osteoarthritis capable of intraarticular or local administration.

### [ADVANTAGEOUS EFFECTS]

When a composition for treating osteoarthritis comprising hydrophilized sulfasalazine and additionally hyaluronic acid is prepared by the preparation method according to the present invention, the dissolution state is maintained even after passing a neutralization step after dissolution, and thereby precipitation by salting out of sulfasalazine occurring after neutralization is prevented. Thus, a composition comprising high concentration of sulfasalazine can be prepared, and the composition prepared accordingly is highly useful for treating osteoarthritis, particularly degenerative osteoarthritis, through intraarticular injection or local administration, since it comprises high concentration of sulfasalazine compared with conventional formulation with low concentration due to poor solubility of sulfasalazine.

### [BRIEF DESCRIPTION OF DRAWINGS]

FIG. 1 is a photograph of composition for osteoarthritis treatment comprising 1, 2, 4, and 8 mmol/l of hydrophilized sulfasalazine and hyaluronic acid according to the present invention.
FIG. 2 is a graph showing the in vitro test result that inhibition of inflammation and cartilage destruction factor expression by composition according to the present invention, in the human adipocyte-derived stem cell in which inflammation is induced with IL-1b.

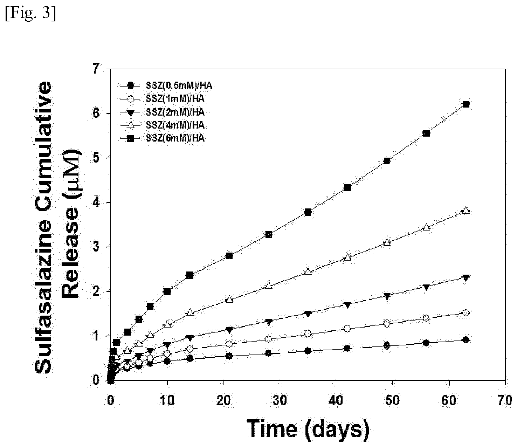

FIG. 3 is a graph measuring the cumulative release of sulfasalazine amount released into saline, when the mixture of hydrophilized sulfasalazine and hyaluronic acid is contained in saline.

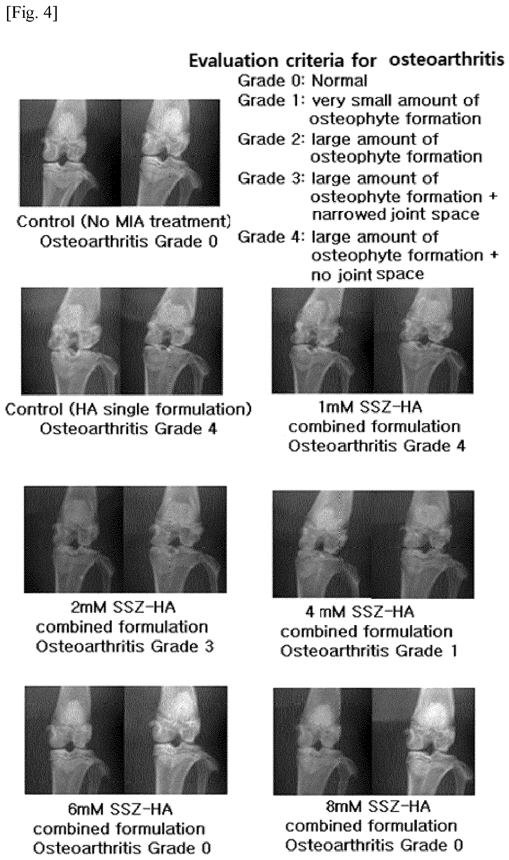

FIG. 4 is X-ray radiographs of osteoarthritis animal model (rat) induced by treating 1mg MIA in synovium at 8 weeks after administering the osteoarthritis composition comprising hydrophilized sulfasalazine and hyaluronic acid according to the present invention of 0 to 8 mmol/l.

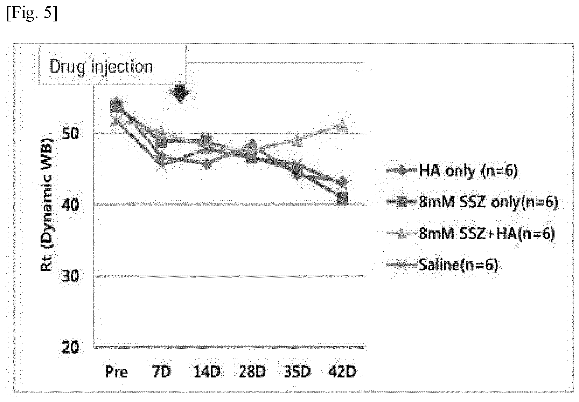

FIG. 5 is the result of administering the osteoarthritis composition comprising hydrophilized sulfasalazine and hyaluronic acid according to the present invention of 0 to 8 mmol/l to the osteoarthritis animal model (rat) induced by treating 1mg MIA in synovium and observing the reduction of symptoms for 6 weeks using equipment capable of measuring symptom levels of animal model.

### [DETAILED DESCRIPTION OF THE EMBODIMENT]

As one aspect to achieve such a purpose, the present invention relates to a composition for treating osteoarthritis capable of intraarticular or local administration, containing 2 to 10 mmol/l of hydrophilized sulfasalazine and 1 to 2 (w/v)% of hyaluronic acid as active ingredients, and a method for improving or treating osteoarthritis by administering the same.

The sulfasalazine (hereinafter, refer to "SSZ") is a compound formed by an azo bond of sulfapyridine and 5-aminosalicylic acid (5-ASA), and has the structure of the following chemical formula 1.

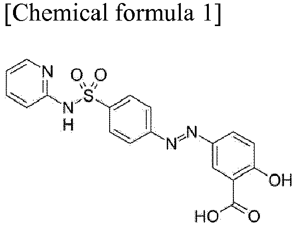

In the present invention, the composition is characterized in that 2 to 10 mmol/l of hydrophilized sulfasalazine as an active ingredient for osteoarthritis treatment is contained.

Herein, the term "hydrophilized sulfasalazine" means one of which the solubility or dispersion uniformity of poorly water-soluble sulfasalazine is increased in an aqueous medium and such a hydrophilized sulfasalazine comprises i) water-soluble salt form of sulfasalazine, or ii) mixture dispersed uniformly by mixing hydrophilization solvent and sulfasalazine in an aqueous medium, and the hydrophilization solvent may be polyethylene glycol (PEG).

The form of i) water-soluble salt of sulfasalazine may be a form of acid addition salt or alkali addition salt by adding hydrochloric acid, sodium chloride, calcium chloride, or potassium chloride to the sulfasalazine, and may be obtained by mixing sulfasalazine with polyethylene glycol.

Preferably, the sulfasalazine of the present invention may comprise the state mixed with ii) polyethylene glycol. The sulfasalazine mixed with polyethylene glycol may be obtained by adding the polyethylene glycol to a pH-adjusted water-soluble medium using a salt and adding the sulfasalazine to the water-soluble medium comprising the polyethylene glycol.

This balanced salt solution may comprise various molecular weight and concentration of polyethylene glycol, for example, it may comprise polyethylene glycol with 380 to 420 of an average molecular weight at the concentration of 1% to 40% (w/v).

In this regard, the present inventor confirmed that the osteoarthritis therapeutic effect of sulfasalazine was shown only at high enough concentrations but not at a concentration lower than the above range, for example, at 1mM. When the sulfasalazine is comprised of less than 2mM (0.08 w/v%), anti-osteoarthritis effect could not be shown, and when it is over 10mM (0.4 w/v%), it is not suitable for liquid formulation or injection formulation due to poor solubility of sulfasalazine itself.

Meanwhile, a hyaluronic acid, its salt or its derivative can be further comprised in the composition with the hydrophilized sulfasalazine described in the present invention.

The hyaluronic acid is a major component of extracellular matrix, known to protect tissues such as iris, retina, etc., and cells such as vascular endothelial cell, epithelial cell, etc., by its mechanical and physiological buffer role, forming an adhesive and elastic solution as widely spread in cellular matrix of the connective tissue under the physiologic condition. In the present invention, the hyaluronic acid has no limit for physical property, form, size, etc., and it is preferable to be aseptically treated, but more preferably, the hyaluronic acid having 1.0 x 10⁶ to 6.0 x 10⁶ g/mol of weight average molecular weight may be used. In addition, the hyaluronic acid may be used as a form of pharmaceutically acceptable salt, the pharmaceutically acceptable salt comprises sodium salt, potassium salt, calcium salt, etc., and preferably comprises sodium salt. In addition, the derivative of hyaluronic acid may also be used, and this derivative may be a chemically modified hyaluronic acid by cross-linking, sulfuration, esterification or amino acid bond. As described above, in the present invention, the concentration of hyaluronic acid is preferable to be 1 to 2 % (wt/vol) of the total composition. When used less than the above range, it may not reside for an appropriate time in the synovial cavity of osteoarthritis due to too low viscoelasticity, and when used more than over the above range, it is difficult to guarantee proper liquidity as a liquid formulation due to excessively high viscoelasticity.

As specific one aspect, the composition for treating osteoarthritis according to the present invention is characterized by being administered intraarticulary or locally. According the experiment of the present inventors, it is necessary that the sulfasalazine is required to be used at a concentration of 2mM or more, since it could not show an effective level of osteoarthritis treatment effect at a low concentration, for example ImM, but there is a high risk of serious side effects when it is orally administered to maintain such a high concentration of sulfasalazine in blood. Thus, the composition for treating osteoarthritis according to the present invention may be formulated as a liquid formulation preferably, and more preferably, an injectable formulation by hydrophilizing the sulfasalazine to be administered intraariculary and locally in an osteoarthritis area. For the formulation, the composition for treating osteoarthritis according to the present invention may further comprise a pharmaceutically acceptable additive in addition to the specific concentration of sulfasalazine and hyaluronic acid. The pharmaceutically acceptable additive means a carrier or diluent which does not inhibit the biological activity and characteristics of active ingredient without significantly stimulating an organism. There is an isotonization agent, buffer, stabilizer, pH regulator, etc. for the pharmaceutically acceptable additive. Moreover, since the composition according to the present invention is preferable to be a liquid formulation, it may further comprise a carrier comprising an aqueous solution for that. The carrier comprising an aqueous solution may comprise one or more kinds of pharmaceutically acceptable carriers selected from the group consisting of distilled water, phosphate buffered saline, balanced salt solution and saline. Then, the content of carrier used may be adjusted according to the amount required for the total capacity of liquid formulation or injection formulation to be prepared. The balanced salt solution means a salt solution providing physiological pH and salt concentration (osmotic pressure), and it may be called equilibrium salt solution, and a common balanced salt solution comprises one or more kinds selected from the group consisting of sodium, potassium, calcium, magnesium and chlorides thereof. Herein, the term "administration" means introducing a component of the present invention to a patient by any appropriate method, and the administration route of the present invention is intraarticular or local injection. Herein, "treatment" means stopping, delaying or improving the progress of disease when used for a subject showing a pathogenic symptom. The treatment method of the present invention comprises administering the hydrophilized sulfasalazine and hyaluronic acid in a therapeutically effective dose. The specific therapeutically effective dose for a certain patient may be vary according to various factors including the kind and level of reaction to be achieved, age, body weight, general health condition, age gender and diet of patient, administration time, treatment period, combinated drug, and similar well-known factors in the medical field.

As another aspect, the present invention relates to a method for preparing a composition for treating osteoarthritis capable of intraarticular or local administration comprising hydrophilized sulfasalazine and hyaluronic acid. As a preferable aspect, the preparation method of a composition for treating osteoarthritis according to the present invention comprises a) a step for preparing PEG aqueous solution of pH 11 to pH 14 by dissolving a basic additive and PEG in water, and dissolving sulfasalazine in it; b) a step for dissolving a hyaluronic acid, its salt or derivative in the product of step a); and c) a step for neutralizing the product prepared in step b) with an acidic additive.

The step a) is a step of hydrophilizing poorly soluble sulfasalazine, and is a step of reacting sulfasalazine with the basic additive and polyethylene glycol (PEG). Specifically, as the basic additive, 0.01 to 1 (w/v)% of NaOH, KOH or ammonia may be used, thereby the pH of PEG aqueous solution becomes 11 or more to 14 or less. Accordingly, the sulfasalazine could be usefully dissolved. When the concentration of basic additive is out of the above range, sulfasalazine is salted out and precipitated during the following neutralization step. Thus, it is not possible to be used as a liquid formulation. For the content of sulfasalazine and the like, details about the composition according to the present invention may be applied as they are. Meanwhile, the polyethylene glycol stabilizes solubilization of sulfasalazine by the property of dissolving both hydrophilic and hydrophobic components, and preferably the polyethylene glycol having 400 to 20,000 of an weight average molecular weight, more preferably, PEG400, 1500, 4,000, 6,000, 20,000 may be used, and PEG may be used at an amount of 1 to 40 (w/v)% of the total composition.

Furthermore, step b) is a step of adding and mixing hyaluronic acid to the dissolved sulfasalazine. The physical property, form, size, and content of hyaluronic acid are the same as defined in the composition according to the present invention, and it is preferable to be aseptically treated. The added hyaluronic acid may be a powder form or liquid solution, and for example, may be a powder form. In addition, it is preferable to mix the hydrophilized sulfasalazine and hyaluronic acid at 10 to 40 °C for 3 hours to 6 hours. When it is mixed at below the temperature range, the components of the composition may not be mixed homogeneously, and mixing at the temperature range is undesirable, since it is difficult to prepare a stable formulation. In addition, when it is mixed less than the time range, the components of composition may be not homogeneously, and when it is mixed over the time, there may be a change in viscosity of hyaluronic acid.

In addition, step c) is a step of neutralizing the product prepared in step b) with an acidic additive to bring the pH to the level being suable for administration to the human body. As long as the acidic additive can adjust an appropriate pH and is harmless to the human body, it may be used without limitation as the acidic additive, for example, acetic acid, hydrochloric acid, phosphoric acid, sulfuric acid, citric acid, nitric acid, and tartartic acid, but not limited thereto.

Meanwhile, the preparation method of composition for treating osteoarthritis may further include a step of adding a pharmaceutically acceptable additive, carrier or salt, or a step of sterilizing for preparing an aseptic formulation.

The method described above allows the present inventors to make a stable composition including sulfasalazine as a concentration sufficiently high to treat osteoarthritis, by solving the problem that a highly concentrated sulfasalazine formulation could not be prepared due to unique poor solubility of sulfasalazine, even though a formulation containing a high concentration of sulfasalazine was required for osteoarthritis treatment in the past. Specifically, since in the preparation method according to the present invention, use of a specific concentration of basic additive prevents precipitation of sulfasalazine via salting out in neutralization step and allows the stable dissolved state, a formulation comprising a high concentration of sulfasalazine can be prepared.

### [MODE FOR INVENTION]

Hereinafter, the present invention will be described in more detail by examples. However, these examples are only for illustrating the present invention, and the scope of the present invention is not limited by these examples.

Hereinafter, the present invention will be described in more detail by examples. However, the following examples are only for illustrating the present invention, and the present invention is not limited by the following examples.

### Example 1: Preparation of mixed composition of 1 to 8 mM hydrophilized sulfasalazine and 1 to 2 (w/v)% hyaluronic acid

A composition comprising 1, 2, 4 and 8 mmol/l of hydrophilized sulfasalazine and 1 to 2 (w/v)% of hyaluronic acid was prepared by the following method.

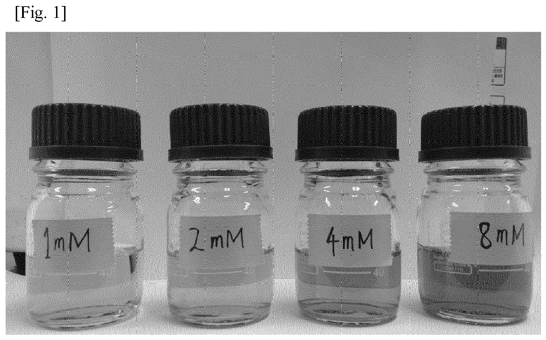

At first, a solution of pH 10 or more was prepared using 1% KOH. After that, 4 (w/v)% of PEG6000 was added to the prepared solution, then the final concentration 1, 2, 4, 8 mmol/l of sulfasalazine was added and stirred for 3 hours. Next, the solution prepared by stirring was filtered with a 0.22 µm sterile filter (Millipore Company Millex-GP), then hyaluronic acid (Ildong hyaluronic acid Ildong raw materials) of final concentration 1% w/vol (10 mg/ml) was added, and it was stirred and mixed. After mixing for 4 hours, 1% hydrochloric acid solution was added to adjust its pH to 7.5, thereby preparing the mixed composition of hydrophilized sulfasalazine and hyaluronic acid (see FIG. 1).

### Example 2: Confirmation test of sustained release of 1 to 6 mM hydrophilized sulfasalazine and 1 to 2 (w/v)% hyaluronic acid

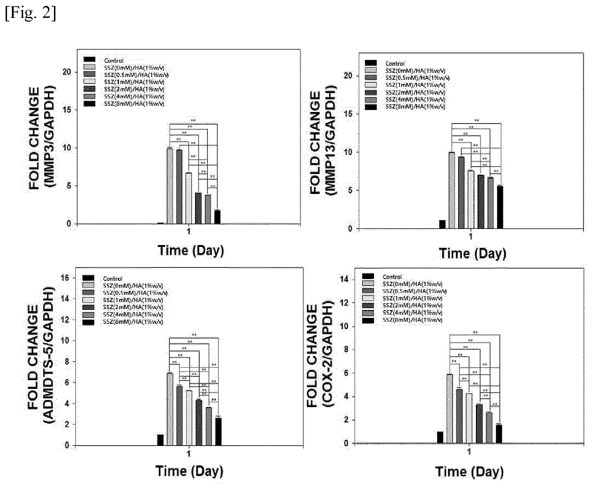

The mixture prepared in Example 1 was contained in saline, and the cumulative release of sulfasalazine to the saline from the sulfasalazine-hyaluronic acid mixture was quantified. As a result, it was confirmed that the release of sulfasalazine mixed with hyaluronic acid was maintained for at least 60 days or more, which showed the sustained release of sulfasalazine (See FIG. 2).

### Example 3: in vitro anti-osteoarthritis efficacy test of 1 to 8 mM hydrophilized sulfasalazine-hyaluronic acid mixture

The effect on the expression of inflammation and cartilage destruction factors (MMP-3, MMP-13, ADMT-5, COX-2) was confirmed with RealTime-PCR from Human Adipose Derived stem cells (ATCC PCS-500-011) which were inflammated with IL-b, followed by treating the mixtures prepared in Example 1. The result, confirmed that the expression of inflammation and cartilage destruction factors was inhibited, the level of inhibitin was directly dependent on the concentration of hydrophilized sulfasalazine.

### Example 4: in vivo anti-osteoarthritis efficacy test for of 1 to 8 mM hydrophilized sulfasalazine-hyaluronic acid mixture

After inducing an osteoarthritis animal model by treating 1mg MIA (Monosodium IodoAcetate) to synovium of 8 week-old rat (Sprague Dawley), after 1 week, mixtures prepared in Example 1 was administered by intraarticular injection. At 8 weeks after administration, the joint region of experimental animals was observed with X-ray. As the result of observation, it was confirmed that while the single formulation of hyaluronic acid did not have any protective effect of cartilage, 1 to 4 mmol/l suldasalazine-hyaluronic acid complex delayed the progress of osteoarthritis compared with the control group, and 6 to 8 mmol/l hydrophilized sulfasalazine-hyaluronic acid complex completely inhibited the progress of osteoarthritis.

### Example 5: Animal model efficacy test for osteoarthritis symptom inhibition of 2 to 8 mM hydrophilized sulfasalazine-hyaluronic acid mixture

After inducing osteoarthritis in animal models by the same method as Example 3, after 1 week, saline, single formulation of hyaluronic acid (HA only), 8 mmol/l hydrophilized sulfasalazine solution (8mM SSZ only) or 8mM hydrophilized sulfasalazine-hyaluronic acid (8mM SSZ-HA) complex prepared in Example 1 was administered by intraarticular injection. The right and left feet weights were respectively measured using Incapacitance tester (Linton instrument Co., UK, Ser No. 01/45/25) or equivalent measurement equipment every week by 4th week after administration. When a leg of rat developed osteoarthritis by MIA, rat stood in the holder of tester leaning on the foot which was not treated with MIA. Each weight (g) of left and right leg was measured and shown in the condition that the abdomen of rat was not touching the sensor of device. The experimental result was represented as mean (%) ± standard error by calculating the weight ratio of right foot to the weight of both feet.

The result indicated that sulfasalazine alone showed no primary pain control effect and single formulation of hyaluronic acid showed the effect only at the beginning, whereas 8mM sulfasalazine-hyaluronic acid mixture showed a uniform pain control effect from the beginning to the end of the test.

## Claims

1. A composition for treating osteoarthritis capable of intraarticular and local administration comprising 2 to 10 mmol/l of hydrophilized sulfasalazine as an active ingredient.

2. The composition for treating osteoarthritis capable of intraarticular and local administration of claim 1, further comprising hyaluronic acid, its salt or derivative at an amount of 1 to 2 (w/v)% of the total composition, in addition to the hydrophilized sulfasalazine.

3. The composition for treating osteoarthritis of claim 2, wherein the derivative of hyaluronic acid is **characterized by** being chemically modified hyaluronic acid by cross-linking, sulfuration, esterification or amino acid bond.

4. The composition for treating osteoarthritis of claim 1, wherein the composition is a liquid formulation.

5. The composition for treating osteoarthritis of claim 1, wherein the composition is an injectable formulation.

6. A preparation method of a composition for treating osteoarthritis capable of intraarticular and local administration comprising,
a) a step of preparing an aqueous solution having pH 11 or more to pH 14 or less by dissolving a basic additive in water, and dissolving sulfasalazine in the aqueous solution;
b) a step of dissolving a hyaluronic acid, its salt or derivative in the product of step a); and
c) a step of neutralizing the product prepared in step b), with an acidic additive.

7. The preparation method of claim 6, wherein the basic additive is one or more selected from the group consisting of sodium hydroxide, potassium hydroxide and ammonia of 0.01 to 1 (w/v)%.

8. The preparation method of claim 6, wherein the aqueous solution further comprises PEG.

9. The preparation method of claim 8, wherein the PEG has an average molecular weight of 400 to 20,000.

10. The preparation method of claim 8, wherein the PEG is 1 to 40 (w/v)%, based on the total composition.

11. A composition for treating osteoarthritis capable of intraarticular and local administration, prepared by the method of any one of claims 6 to 10.

12. A method for improvement or treatment of osteoarthritis, comprising a step of administering a composition comprising 2 to 10 mmol/l of hydrophilized sulfasalazine as an active ingredient.

13. The method for improvement or treatment of osteoarthritis of claim 12, wherein the composition is **characterized by** intraarticular or local administration.
